# FASCICULE DE BREVET EUROPEEN

(11) **EP 0 954 347 B1**
(45) Date de publication et mention de la délivrance du brevet: **20.08.2003**
(21) Numéro de dépôt: 97952091.3
(22) Date de dépôt: 17.12.1997
(51) Int. Cl.: A61M 5/50, A61M 5/28, A61M 5/32

(54) **SERINGUE D'INJECTION A AIGUILLE FAISANT SAILLIE DANS LE CORPS DE SERINGUE**
INJEKTIONSSPRITZE MIT EINER INS INNERE DES SPRITZENKÖRPERS HINEINRAGENDEN NADEL
INJECTION SYRINGE WITH NEEDLE PROJECTING IN THE SYRINGE BODY

(30) Priorité: 17.12.1996 FR 9615507
(43) Date de publication de la demande: 10.11.1999
(73) Titulaire: SEDAT, 69540 Irigny (Rhône) (FR)
(72) Inventeur: ARNISSOLLE, Yves, F-69230 Saint Genis Laval (FR)
(74) Mandataire: Blot, Philippe Robert Emile
(86) Numéro de dépôt international: FR9702333
(87) Numéro de publication internationale: WO98026825

(56) Documents cités:
- FR-A- 2 638 091
- US-A- 2 460 039
- US-A- 5 370 628

## Description

La présente invention concerne une seringue d'injection du type comportant, d'une part un corps de seringue allongé comprenant un tube et une paroi avant munie d'une aiguille d'injection, et d'autre part un piston arrière d'actionnement monté déplaçable dans le tube, et dans laquelle l'aiguille d'injection se prolonge axialement à l'intérieur du corps de seringue jusqu'à une extrémité arrière, à l'écart de laquelle est initialement disposée, dans le corps de seringue, au moins une paroi transversale perforable, l'aiguille et la paroi transversale étant déplaçables l'une par rapport à l'autre depuis une position initiale dans laquelle la paroi transversale est écartée de l'aiguille jusqu'à une position finale dans laquelle la paroi transversale est transpercée par ladite aiguille.

La paroi transversale perforable est, par exemple, portée par le piston d'actionnement.

En variante, la paroi transversale est par exemple formée par un piston intermédiaire disposé dans le corps de seringue entre l'extrémité arrière de l'aiguille et le piston d'actionnement. Le piston intermédiaire délimite alors avec le piston d'actionnement un logement dans lequel est contenu le fluide à injecter.

Dans ce dernier cas, lors de l'enfoncement du piston d'actionnement, le piston intermédiaire est perforé par l'extrémité arrière de l'aiguille afin de permettre l'écoulement du fluide au travers de l'aiguille.

Une telle seringue est décrite par exemple dans le document US-5.370.628.

Si le tronçon de l'aiguille faisant saillie à l'intérieur du corps de seringue est long, le piston intermédiaire risque d'être perforé avec un léger décalage angulaire et/ou axial par rapport à l'axe du corps de seringue, du fait de la faible rigidité de l'aiguille.

Dans ces conditions, en fin d'injection, lorsque le piston d'actionnement entre en contact avec le piston intermédiaire et sollicite celui-ci vers l'avant en vue de son déplacement suivant le tronçon intérieur de l'aiguille, le décalage existant entre l'axe de l'aiguille et l'axe de la seringue rend difficile le déplacement du piston intermédiaire le long de celle-ci et risque de conduire à un blocage.

Des problèmes analogues sont rencontrés lorsque la paroi transversale est portée par l'extrémité avant du piston d'actionnement et constitue la face avant de celui-ci.

L'invention a pour but d'apporter une solution aux dysfonctionnements de la seringue résultant d'une perforation décalée angulairement et/ou axialement de la paroi transversale par l'extrémité arrière de l'aiguille.

A cet effet, l'invention a pour objet une seringue d'injection du type précité caractérisée en ce qu'elle comporte des moyens rigides de guidage axial de l'extrémité arrière de l'aiguille lors de la perforation de la paroi transversale.

Les moyens rigides de guidage axial de l'extrémité arrière de l'aiguille assurent un maintien en position de la partie de l'aiguille faisant saillie à l'intérieur du corps de seringue, de sorte que celle-ci perfore la paroi transversale suivant l'axe commun de l'aiguille de la seringue, évitant les problèmes de blocage mentionnés ci-dessus.

L'invention a en outre pour objet un ensemble porte-aiguille comportant une paroi solidaire d'une aiguille d'injection traversant ladite paroi, ainsi qu'un protecteur mobile de l'extrémité d'injection de l'aiguille, déplaçable par rapport à l'aiguille entre une position escamotée en retrait de l'extrémité d'injection de l'aiguille et une position active de protection dans laquelle l'extrémité avant du protecteur se trouve en avant de l'extrémité d'injection de l'aiguille, lequel protecteur comporte des jambages traversant ladite paroi et s'étendant le long de ladite aiguille, caractérisé en ce qu'il comporte un organe rigide de guidage axial de l'extrémité arrière de l'aiguille, lequel organe rigide de guidage axial est porté par les extrémités libres des jambages du protecteur.

L'invention sera mieux comprise à la lecture de la description qui va suivre, donnée uniquement à titre d'exemple et faite en se référant aux dessins sur lesquels :
- la figure 1 *est* une vue en coupe longitudinale d'une seringue selon l'invention, avant utilisation ;
- la figure 2 est une vue de détail à plus grande échelle de la zone entourant l'extrémité arrière de l'aiguille de la seringue de la figure 1 ;
- les figures 3 à 6 sont des vues en coupe longitudinale de la seringue de la figure 1 réalisées à différents stades successifs d'utilisation de la seringue, et
- La figure 7 est une vue de détail à plus grande échelle de la zone entourant la paroi avant de la seringue après la fin de l'injection.

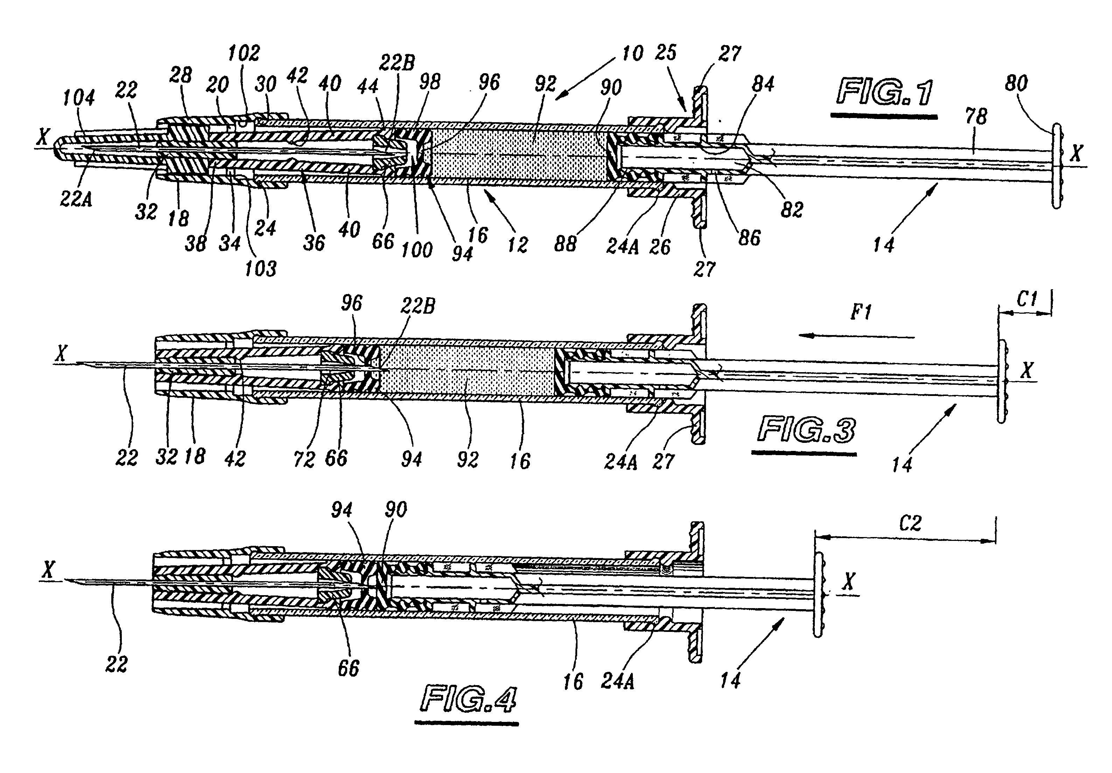

La seringue d'injection 10 représentée sur la figure 1, de forme générale de révolution d'axe X-X, est une seringue à usage unique. Elle est proposée prête à l'emploi et contenant déjà un fluide médical à injecter. Elle comporte essentiellement un corps de seringue 12 allongé et un piston arrière d'actionnement 14 monté déplaçable à l'intérieur du corps 12.

Le corps de seringue 12 est formé d'un tube 16 à l'extrémité avant duquel est fixé un porte-aiguille 18 portant une paroi avant 20 du corps de seringue. Cette paroi avant 20 est munie d'une aiguille d'injection traversante 22. Celle-ci comporte une extrémité avant d'injection 22A faisant saillie par rapport au corps. L'aiguille se prolonge axialement à l'intérieur du corps 12 jusqu'à une extrémité arrière ou proximale 22B. Le tronçon de l'aiguille s'étendant dans le corps de seringue est plus long que le tronçon de l'aiguille faisant saillie en dehors du corps de seringue.

Le tube 16 est réalisé par exemple en verre et a une section circulaire. Son extrémité avant est munie extérieurement d'un bourrelet périphérique 24 de maintien du porte-aiguille 18. De même, à son extrémité arrière, le tube 16 comporte extérieurement un bourrelet périphérique 24A. A cette extrémité arrière est rapporté un organe de préhension 25 facilitant la préhension du corps de seringue entre l'index et le majeur. Celui-ci comporte un manchon 26 encliqueté extérieurement à l'extrémité du tube 16 sur le bourrelet 24A et deux pattes 27 diamétralement opposées pour l'appui des doigts.

Le porte-aiguille 18 est délimité extérieurement par un manchon 28. La paroi avant 20 est venue de matière avec le manchon 28 et s'étend transversalement en un emplacement intermédiaire de celui-ci. Sur la paroi intérieure du manchon 28 est ménagée, légèrement en arrière de la paroi avant 20, une gorge périphérique 30 de réception du bourrelet 24.

La paroi avant 20 présente un plot axial 32 venu de matière, de fixation de l'aiguille d'injection 22. Ce plot est dirigé vers l'extrémité d'injection 22A de l'aiguille et est reçu à l'intérieur de l'espace délimité par le manchon 28.

Trois ouvertures 34 identiques sont ménagées au travers de la paroi avant 20. Elles sont régulièrement réparties angulairement autour du plot 28 sur un même contour circulaire, et présentent une forme arquée. Les figures sont des coupes longitudinales de la seringue effectuées de part et d'autre de l'axe X-X dans la partie médiane de deux des ouvertures 34.

Ces ouvertures 34 assurent le passage et le guidage d'un protecteur d'aiguille 36. Celui-ci comporte à l'avant une bague de protection 38 en matière plastique rigide, dont les diamètres intérieur et extérieur sont adaptés afin que la bague 38 se loge dans l'espace annulaire défini entre le plot 32 et le manchon 18. Cette bague est prolongée par trois jambages 40 identiques élastiquement déformables et espacés angulairement de 120°. Ces jambages 40 présentent en section une légère courbure correspondant à celle de la bague 38 et ont une longueur sensiblement égale à la longueur du tronçon de l'aiguille 22 reçu dans le corps de seringue 12.

Par ailleurs, chaque jambage 40 comporte sur toute sa largeur un premier bombage intérieur 42, disposé légèrement en arrière de la bague 38, et un second bombage extérieur 44 disposé à son extrémité libre.

En outre, comme représenté sur la figure 2, les jambages 40 comportent au voisinage de leurs extrémités libres un chambrage intérieur 46 à fond cylindrique s'étendant sur toute la largeur de chaque jambage. Ce chambrage 46 est délimité sur chaque jambage à l'avant par un épaulement 48 ménagé dans l'épaisseur du jambage et à l'arrière par une saillie 50 ménagée au niveau du bombage 44. Chaque saillie 50 porte une surface de came 52 formée par une surface courbe sensiblement circulaire reliant le sommet arrondi de la saillie 50 au fond du chambrage 46.

Des moyens 64 de guidage axial de l'extrémité arrière 22B de l'aiguille sont retenus entre les extrémités libres des jambages 40. Ces moyens comportent un organe 66 de révolution maintenu entre les jambages 40. Cet organe est formé dans un matériau rigide, notamment une matière plastique rigide. Il comporte extérieurement, à l'arrière, une paroi latérale tronconique 68. Il est bordé extérieurement, à l'avant, par une collerette 70. La surface latérale cylindrique de la collerette 70 est creusée dans son milieu en considérant la direction de l'axe X-X par une gorge périphérique 71 peu profonde. La profondeur de la gorge est en particulier inférieure à la hauteur des bombages 44. Avant utilisation de la seringue (figures 1 et 2), la collerette 70 est reçue dans le chambrage 46 et sa surface latérale s'applique sur le fond cylindrique du chambrage 46. Les bombages 44 des jambages sont alors plaqués contre la surface intérieure du tube 16. Ainsi, l'organe 66 est maintenu centré suivant l'axe X-X par coopération avec la paroi du corps de la seringue.

L'organe 66 comporte un passage axial 72 traversant celui-ci de part en part. Ce passage comporte à l'avant un tronçon cylindrique 74 prolongé par un tronçon 76 de section progressivement décroissante vers l'arrière. Ce tronçon 76 est défini par une surface tronconique dont la section, à son extrémité la plus étroite, est sensiblement égale à celle de l'aiguille 22.

Comme représenté sur la figure 2, avant utilisation de la seringue, l'extrémité arrière 22 de l'aiguille s'étend dans le passage 72, de sorte que l'extrémité de l'aiguille est complètement recouverte par l'organe 66.

Le piston arrière 14 comporte un poussoir allongé 78 ayant une section en croix et présente à son extrémité arrière une pastille 80 d'appui du pouce de l'opérateur. A son extrémité opposée est prévu axialement un logement 82 ouvert sur l'avant, servant à la réception de l'extrémité arrière 22B de l'aiguille en fin d'injection. Ce logement 82, de forme allongée suivant l'axe X-X, a une section circulaire. Il est délimité par une paroi cylindrique 84 munie d'un évent calibré 86.

La paroi 84 présente extérieurement à son extrémité avant un bourrelet hélicoïdal 88 de fixation par emboîtement d'une membrane d'extrémité 90 en forme de cuvette. Cette membrane obture l'ouverture principale avant du logement 82, et constitue une paroi transversale adaptée pour coulisser de manière étanche à l'intérieur du tube 16.

Tel que représenté sur la figure 1, le fluide à injecter 92 est disposé à l'intérieur du tube 16 dans un espace délimité par la membrane 90 du piston arrière et un piston intermédiaire 94. Le piston intermédiaire 94 comporte une paroi transversale perforable 96, entourée par un manchon latéral 98 venu de matière et muni de nervures périphériques extérieures afin d'assurer l'étanchéité au liquide et au gaz entre celui-ci et la paroi latérale intérieure du tube 16. Le piston intermédiaire 94 est appliqué initialement contre l'extrémité du protecteur d'aiguille. La paroi 96 et le manchon 98 délimitent à l'avant une cuvette 100 dans laquelle est partiellement reçue l'extrémité arrière de l'organe 66 qui fait saillie par rapport aux jambages 40.

L'extrémité avant du tube 16 est emmanchée dans le manchon 28 et y est retenue par encliquetage. La gorge périphérique 30 étant prévue légèrement en retrait par rapport à la paroi transversale 20, cette dernière délimite avec l'extrémité avant du tube 16 un canal annulaire 102, disposé immédiatement en arrière de la paroi 20 et dont le fond est formé par le manchon 28. La largeur du canal 102 mesurée suivant l'axe X-X est sensiblement égale au double de la largeur des bombages 44 mesurée suivant ce même axe. A l'avant, le canal 102 est délimité par un épaulement 103 ménagé dans l'épaisseur du manchon 18. La profondeur du creux formé par le canal 102 est sensiblement égale à la hauteur des saillies formées par les bombages 44.

Par ailleurs, un capuchon de protection 104 de l'aiguille 22 est emmanché à l'intérieur du manchon 28 et recouvre l'extrémité d'injection 22A de l'aiguille.

Le montage de la seringue s'effectue de la manière suivante.

Le porte-aiguille 18 est collé sur l'aiguille 22. Le protecteur 36 est monté au travers de la paroi avant par engagement des jambages 40 dans les passages 34. Il est disposé de telle sorte que la bague de protection avant 38 entoure le plot 32 et que les jambages 40 s'étendent le long de la partie arrière de l'aiguille 22. L'organe 66 est ensuite mis en place dans le chambrage 46 par déformation élastique de l'extrémité des jambages 40. L'organe 66 est maintenu en position par l'élasticité des jambages 40 dont les extrémités libres tendent par construction à se rapprocher de l'extrémité 22B de l'aiguille. Le capuchon avant 104 est alors mis en place par emmanchement de celui-ci dans le manchon 28.

Le porte-aiguille 18, ainsi muni du capuchon 104 et de l'organe 66, peut être manipulé sans risque de dégradations des extrémités de l'aiguille, celle-ci étant protégée à ses deux extrémités. En particulier, il peut être distribué sur les chaînes de fabrication dans des bols vibrants.

Parallèlement à l'assemblage du porte-aiguille, le tube 16 est muni de l'organe de préhension 25. Il est rempli du fluide 92 disposé entre le piston intermédiaire 94 et le piston d'actionnement 14. Le porte-aiguille 18 est mis en place par encliquetage à l'extrémité avant du tube 16, comme représenté sur la figure 1.

L'introduction des jambages 40 dans le tube 16 lors du montage du porte-aiguille sur le tube est aisée puisque les jambages 40 associés à l'organe 66 forment un ensemble unitaire et cohérent dont le diamètre extérieur (diamètre mesuré au niveau des bombages 44) correspond exactement au diamètre intérieur du tube 16.

Afin de procéder à l'injection, l'opérateur retire le capuchon 104. De manière classique, l'opérateur exerce alors avec le pouce une poussée sur le piston arrière 14 suivant le sens de la flèche F1, en prenant appui sous les pattes 27 avec l'index et le majeur.

La pression ainsi exercée, transmise par l'intermédiaire du liquide 92 au piston intermédiaire 94, provoque le déplacement de celui-ci suivant une course notée C1 vers l'extrémité proximale 22B de l'aiguille. Cette dernière perfore la paroi 96 du piston intermédiaire au cours du mouvement de celui-ci. Le déplacement du piston intermédiaire 94 s'accompagne du déplacement vers l'avant du protecteur d'aiguille 36.

Lors de l'enfoncement, l'organe 66 est entraîné vers l'avant par le protecteur d'aiguille 36 dans le chambrage 46 duquel il est maintenu. En effet, les jambages 40 sont appliqués suivant les bombages 44 sur la paroi latérale du tube 16 retenant ainsi l'organe 66 dans le chambrage 46.

De plus, lors du mouvement de l'organe 66, la paroi 16 assure, par l'intermédiaire des jambages 40, un centrage et un guidage axial de celui-ci afin de garantir son déplacement rigoureusement suivant l'axe X-X.

Lors du mouvement conjoint du piston intermédiaire 94 et de l'organe 66, l'extrémité arrière 22B de l'aiguille entre d'abord en contact avec la surface tronconique 76. Cette dernière assure, au fur et à mesure du déplacement de l'organe 66, un centrage progressif de l'extrémité 22B de l'aiguille. Elle procure en outre un guidage axial de l'aiguille. Ainsi, lorsque l'extrémité arrière 22B de l'aiguille sort de l'organe 66, le tronçon de l'aiguille disposé à l'intérieur du corps s'étend rigoureusement suivant l'axe X-X. Il y est maintenu par l'extrémité de section réduite du passage 72. L'extrémité arrière 22B de l'aiguille perfore ainsi la paroi 96 en étant toujours retenue latéralement par l'organe 66 disposé légèrement en avant sur l'aiguille.

Dans ces conditions, la perforation du piston intermédiaire 94 s'effectue axialement et dans la partie centrale de celui-ci, garantissant ainsi un déplacement ultérieur aisé du piston 94 sur tout le tronçon intérieur de l'aiguille 22.

Le déplacement du piston intermédiaire 94 empalé sur l'aiguille 22 est stoppé à l'issue de la course C1 lorsque les bombages 42 viennent en butée sur le plot 32, comme cela est représenté sur la figure 3. A cet effet, les bombages 42 sont dimensionnés de telle sorte que le protecteur 36 soit maintenu dans sa position rétractée de la figure 3 dans laquelle la bague 38 s'étend dans le manchon 18 malgré la pression exercée par le piston intermédiaire.

La course C1 correspond à la purge de la seringue. En effet, le déplacement du piston arrière 14 alors que le piston intermédiaire 94 est perforé par l'aiguille assure l'évacuation de l'air contenu dans l'aiguille et l'écoulement d'une faible quantité du fluide 92 au travers de celle-ci.

Après cette purge, l'extrémité d'injection 22A de l'aiguille est introduite dans les tissus du patient.

Le fluide 92 est alors injecté au travers de l'aiguille 22 sous l'effet de la poussée du piston arrière 14 enfoncé suivant une course C2 jusqu'à la position représentée sur la figure 4. Dans cette position, l'essentiel du fluide 92 est injecté et la membrane 90 vient au contact de la surface arrière du piston intermédiaire 94.

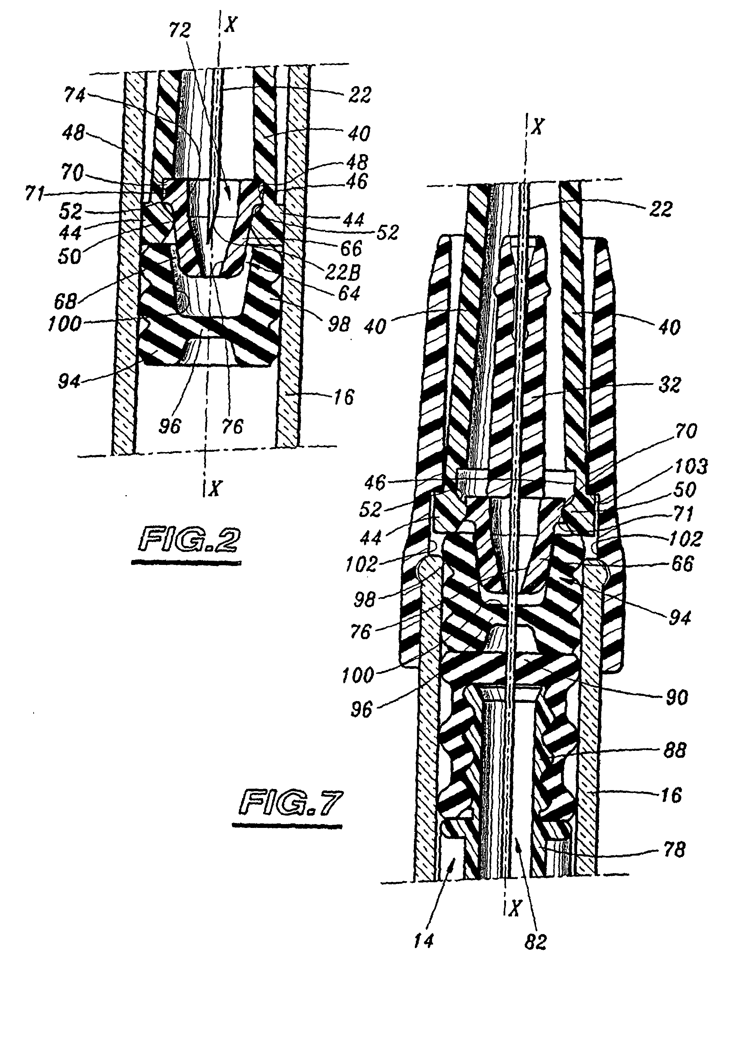

La pression continue exercée par l'opérateur sur le piston arrière 14 provoque ensuite le dégagement des bombages 42 par déformation élastique des jambages 40. Il s'ensuit le déplacement vers l'avant du protecteur 36. Lorsque la bague 38 entre en contact avec la peau du patient, la course du protecteur est stoppée et la poursuite du rapprochement suivant une course C3 du pouce appuyé sur la pastille 80 et des autres doigts maintenus contre les pattes 27 provoque la remontée du corps de seringue 12 dans le sens de la flèche F2 (figure 5).

On comprend que la remontée du corps 12 provoque l'extraction de l'aiguille 22 d'injection du corps du patient.

Il est également possible par simple traction sur le corps de seringue d'extraire l'aiguille des tissus du patient alors que la seringue est dans la position représentée sur la figure 4. L'enfoncement du piston arrière 14 dans le corps de seringue est alors poursuivi avec la seringue dégagée de tout contact avec le patient.

Par ailleurs, l'extrémité arrière 22B de l'aiguille transperce la membrane 90 et est reçue dans le logement 82. Du fait de la présence de l'organe 66, la perforation de la membrane 90 s'effectue rigoureusement suivant l'axe X-X.

Dans la position représentée sur la figure 5, les bombages 44 portés extérieurement par l'extrémité libre des jambages 40 sont disposés en regard du canal annulaire 102. De plus, dans cette position, l'organe 66 s'appuie en butée suivant sa face avant sur le plot 32 et la face avant 20. Il est ainsi immobilisé axialement.

Lors de l'ultime phase d'enfoncement du piston 14 suivant une course C4, le protecteur d'aiguille 36 est amené à se déplacer vers l'avant sous l'action de la poussée du piston arrière 14 transmise par le piston intermédiaire 94 qui est toujours en contact avec l'extrémité libre des jambages 44. A cet effet, l'extrémité arrière de l'organe 66 qui fait saillie par rapport aux jambages 40 s'escamote dans la cuvette 100 permettant ainsi le coulissement du piston intermédiaire 94 malgré l'immobilisation de l'organe 66.

Alors que l'organe 66 est immobilisé, les surfaces de cames 52, ménagées à l'arrière du chambrage 46 coopèrent avec la collerette 70 formant contre-came pour provoquer l'écartement des extrémités libres des jambages. Suite à cet écartement, les bombages 44 sont reçus dans le canal annulaire 102, comme cela est représenté à plus grande échelle sur la figure 7.

Dans cette position, les saillies 50 bordant à l'arrière le chambrage 46 reposent sur la surface latérale de la collerette 70 assurant ainsi une retenue positive des bombages 44 à l'intérieur du canal annulaire 102 et un blocage positif du protecteur d'aiguille 36.

De plus, les saillies 50 sont reçues dans la gorge périphérique 71 de la collerette assurant ainsi une solidarisation axiale de l'organe 66 et du protecteur d'aiguille 36. Ainsi, même si le protecteur d'aiguille est amené à se déplacer légèrement axialement (suivant la longueur du canal annulaire 102), l'organe 66 est entraîné et reste en regard des bombages 44, ce qui permet la retenue positive permanente des bombages 44 dans le canal 102. Ainsi, les extrémités des jambages 40 ne peuvent pas être libérées, ce qui garantit le verrouillage du protecteur d'aiguille 36 dans sa position active de protection.

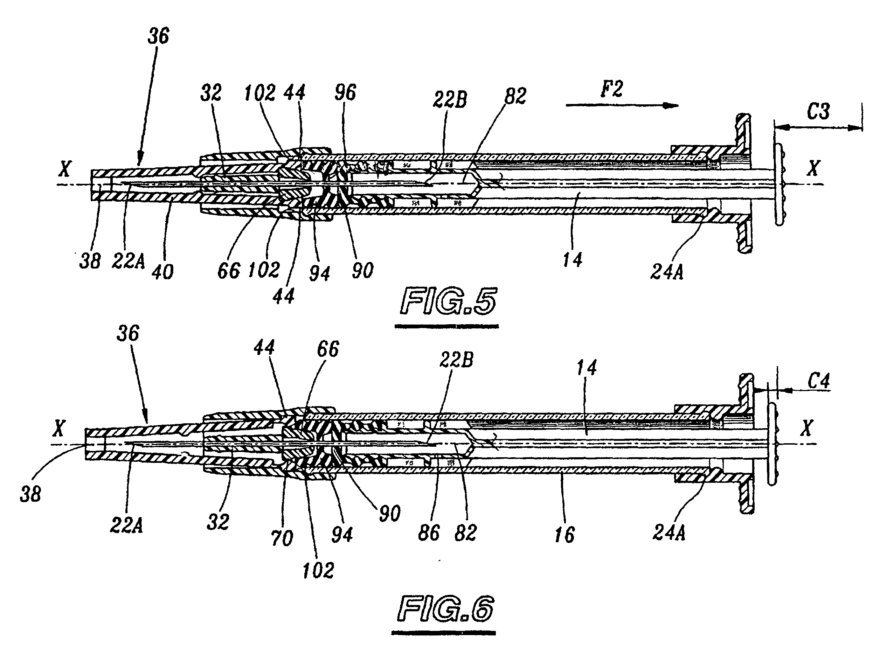

Sur les figures 6 et 7, le protecteur 36 est en position active de protection et s'étend autour de l'extrémité d'injection 22A de l'aiguille. La face avant de la bague de protection 38 est ainsi située légèrement en avant de l'extrémité 22A de l'aiguille d'injection, interdisant tout contact de celle-ci par un élément extérieur et évitant ainsi tout risque de piqûre contaminante pour l'opérateur.

On comprend que les bombages 44 engagés dans le canal 102 entre l'épaulement 103 et l'extrémité du tube 16 maintiennent solidement le protecteur 36 en position de protection, interdisant ainsi tout escamotage accidentel. Ce maintien est en effet garanti par la présence de l'organe 66 dont la collerette 70 retient écartées les extrémités des jambages 40.

La membrane 90 ayant été perforée par l'extrémité arrière 22B de l'aiguille, la seringue est rendue impropre à une nouvelle utilisation. En effet, cette perforation interdit l'effet d'aspiration obtenu normalement à l'intérieur du corps lors du recul du piston arrière, du fait de la présence de l'évent 86. De même, le piston arrière perforé ne permet pas l'expulsion par l'aiguille d'injection 22 d'un éventuel liquide réintroduit dans le corps de seringue.

On comprend que dans une seringue telle que décrite ici, l'organe 66 garantit une perforation correcte du piston intermédiaire 94. En effet, l'extrémité 22B de l'aiguille guidée par la surface tronconique 76 pénètre au centre du piston 96 et suivant l'axe X-X de la seringue.

Dans l'exemple décrit, l'organe 66 est initialement disposé autour de l'extrémité 22B. Toutefois, celui-ci peut être disposé sur l'aiguille légèrement en avant de son extrémité arrière, tout en exerçant correctement sa fonction de guidage de l'extrémité arrière de l'aiguille.

Par ailleurs, dans le cas d'une seringue dans laquelle le porte-aiguille 18 est muni de moyens d'étanchéité entre la paroi 20 et les jambages 40, le piston intermédiaire 94 est supprimé. Toutefois, l'organe 66 est avantageusement maintenu afin d'assurer, dans la position analogue à celle de la figure 4, une perforation de la paroi transversale 90 du piston arrière rigoureusement suivant l'axe X-X de la seringue, garantissant ainsi une pénétration axiale correcte de l'aiguille dans le logement 82.

En variante non représentée, les éléments en saillie formés par les bombages 44 et l'élément complémentaire en creux associé formé par le canal annulaire 102 sont inversés. Ainsi, les jambages comportent extérieurement des creux alors que le corps de seringue comporte des saillies complémentaires en vue du maintien du protecteur dans sa position active de protection.

Dans cette variante également, l'organe 66 assure une retenue positive de l'engagement des reliefs associés en saillie et en creux lorsque le protecteur d'aiguille est dans sa position active de protection.

## Revendications

1. Seringue d'injection (10) comportant d'une part un corps de seringue allongé (12) comprenant un tube (16) et une paroi avant (20) munie d'une aiguille d'injection (22), et d'autre part un piston arrière d'actionnement (14) monté déplaçable dans le tube (16), et dans laquelle l'aiguille d'injection (22) se prolonge axialement à l'intérieur du corps de seringue (12) jusqu'à une extrémité arrière (22B), à l'écart de laquelle est initialement disposée, dans le corps de seringue (12), au moins une paroi transversale perforable (90, 96), l'aiguille (22) et ladite paroi transversale (90, 96) étant déplaçables l'une par rapport à l'autre depuis une position initiale dans laquelle la paroi transversale (90, 96) est écartée de l'aiguille (22) jusqu'à une position finale dans laquelle la paroi transversale (90, 96) est transpercée par ladite aiguille (22), la seringue comportant un protecteur mobile (36) de l'extrémité d'injection (22A) de l'aiguille, déplaçable par rapport au corps (12) sous l'effet de l'enfoncement du piston d'actionnement (14) dans le corps (12), entre une position escamotée dans le corps en retrait de l'extrémité d'injection (22A) de l'aiguille et une position active de protection dans laquelle l'extrémité avant du protecteur se trouve en avant de l'extrémité d'injection (22A) de l'aiguille, lequel protecteur comporte des jambages (40) traversant le corps (12) de seringue et s'étendant partiellement dans le corps (12) le long de ladite aiguille (22), **caractérisée en ce qu'**elle comporte un organe rigide (64) de guidage axial de l'extrémité arrière (22) de l'aiguille lors de la perforation de la paroi transversale (90, 96), lequel organe rigide (64) de guidage axial est porté par les extrémités libres des jambages (40) du protecteur.

2. Seringue selon la revendication 1, **caractérisée en ce que** ledit organe rigide (64) de guidage axial comporte un passage (72) de guidage de l'extrémité arrière (22B) de l'aiguille, la section dudit passage (72) étant sur au moins une partie de sa longueur progressivement décroissante vers ladite paroi transversale (90, 96).

3. Seringue selon la revendication 2, **caractérisée en ce que** l'extrémité arrière (22B) de l'aiguille est initialement disposée dans ledit passage (72).

4. Seringue selon l'une quelconque des revendications précédentes, **caractérisée en ce que** ledit organe de guidage (66) comportant des moyens (44, 46, 70) de centrage dudit organe par rapport à l'axe (X-X) de l'aiguille (22), lesquels moyens de centrage coopèrent initialement avec la paroi latérale intérieure du corps de seringue (12) au voisinage de l'extrémité arrière (22B) de l'aiguille.

5. Seringue selon la revendication 4, **caractérisée en ce que** ledit organe de guidage (66) est monté déplaçable le long de l'aiguille (22).

6. Seringue selon l'une quelconque des revendications précédentes, **caractérisée en ce que** l'extrémité arrière de l'aiguille est complètement recouverte par ledit organe rigide de guidage (64) et est en retrait de celui-ci.

7. Seringue selon les revendication 5 et 6 prises ensemble, **caractérisée en ce que** les jambages (40) comportent extérieurement à leur extrémité libre des saillies (44) adaptées pour être reçues dans un creux (102) ménagé dans la paroi latérale du corps (12) lorsque le protecteur (36) est en position active de protection, l'organe de guidage (66) étant disposé entre les jambages (40) du protecteur et déplaçable avec celui-ci jusqu'à une position d'immobilisation en butée contre le corps de seringue, et **en ce que** les jambages (40) comportent des surfaces de cames (52), coopérant avec l'organe de guidage (66) lorsque celui-ci est immobilisé, pour écarter les extrémités des jambages (40) par déplacement axial du protecteur d'aiguille (36) lors de la phase ultime de l'enfoncement du piston d'actionnement (14) dans le corps (12), l'organe de guidage (66) assurant une retenue positive des saillies (44) dans le creux (102).

8. Seringue selon l'une quelconque des revendications précédentes, **caractérisée en ce que** ledit protecteur (36) et ledit corps de seringue (12) comportant des reliefs associés (44, 102) en saillie et en creux de maintien du protecteur (36) dans sa position active de protection, et **en ce que** ledit organe rigide de guidage axial (64) comportent des moyens (66) de retenue positive de l'engagement desdits reliefs associés (44, 102) en saillie et en creux lorsque le protecteur (36) est dans sa position active de protection.

9. Seringue selon l'une quelconque des revendications précédentes, **caractérisée en ce que** la paroi transversale (90) est portée par le piston d'actionnement (14).

10. Seringue selon l'une quelconque des revendications 1 à 9, **caractérisée en ce que** la ou une paroi transversale (96) est portée par un piston intermédiaire perforable (94) disposé entre l'extrémité arrière (22B) de l'aiguille et le piston d'actionnement (14), le piston intermédiaire (94) délimitant avec le piston d'actionnement (14) un logement pour un fluide (92) à injecter.

11. Ensemble porte-aiguille comportant une paroi (20) solidaire d'une aiguille d'injection (22) traversant ladite paroi (20), ainsi qu'un protecteur mobile (36) de l'extrémite d'injection (22A) de l'aiguille, déplaçable par rapport à l'aiguille entre une position escamotée en retrait de l'extrémité d'injection (22A) de l'aiguille et une position active de protection dans laquelle l'extrémité avant du protecteur (36) se trouve en avant de l'extrémité d'injection de l'aiguille, lequel protecteur comporte des jambages (40) traversant ladite paroi (20) et s'étendant le long de ladite aiguille (22), **caractérisé en ce qu'**il comporte un organe rigide (64) de guidage axial de l'extrémité arrière de l'aiguille, lequel organe rigide (64) de guidage axial est porté par les extrémités libres des jambages (40) du protecteur.

12. Ensemble porte-aiguille selon la revendication 11, **caractérisé en ce que** l'extrémité arrière de l'aiguille est complètement recouverte par ledit organe rigide de guidage (64) et est en retrait de celui-ci.

## Patentansprüche

1. Injektionsspritze (10), welche zu einem Teil aus einem verlängerten Spritzenkörper (12) besteht, der ein Rohr (16) und eine mit einer Injektionsnadel (22) versehene Vorderwand (20) umfasst, und welche zu einem anderen Teil einen hinten liegenden Betätigungskolben (14) aufweist, welcher in dem Rohr (16) verschiebegeführt angeordnet ist, und in welcher sich die Injektionsnadel (22) axial in das Innere des Spritzenkörpers (12) bis zu einem hinteren Ende (22B) ausdehnt, und zu welcher innerhalb des Spritzenkörpers (12) mindestens eine durchstoßbare Querwand (90, 96) anfänglich in einem Abstand angeordnet ist, und die Nadel (22) und die Querwand (90, 96) untereinander aus einer Anfangsposition, in welcher die Querwand (90, 96) von der Nadel (22) beabstandet ist, bis zu einer Endposition, in welcher die Querwand (90, 96) von der Nadel (22) durchdrungen ist, verschiebbar angeordnet sind, und die Spritze ein bewegliches Schutzelement (36) für das Injektionsende (22A) der Nadel beinhaltet, welches in Bezug auf den Spritzenkörper (12) unter Einwirkung des Einschiebens des Betätigungskolbens (14) in den Spritzenkörper (12) zwischen einer in den Spritzenkörper (12) eingeschobenen Position bei zurückliegendem Injektionsende (22A) der Nadel und einer aktiven Schutzstellung, in welcher sich das vordere Ende des Schutzelementes vor dem Injektionsende (22A) der Nadel befindet, verschiebbar angeordnet ist, und dieses Schutzelement Stützelemente (40) aufweist, welche den Spritzenkörper (12) durchdringen und sich zum Teil in den Spritzenkörper (12) längs der Nadel (22) erstrecken, **dadurch gekennzeichnet, dass** sie einen starren Körper (64) zur axialen Führung des hinteren Endes (22B) der Nadel während des Durchstoßens der Querwand (90, 96) aufweist, und dass dieser starre Körper (64) zur axialen Führung von den freien Enden der Stützelemente (40) des Schutzelementes getragen wird.

2. Injektionsspritze nach Anspruch 1, **dadurch gekennzeichnet, dass** der starre Körper (64) zur axialen Führung einen Durchgang (72) zur Führung des hinteren Endes (22B) der Nadel aufweist, wobei der Querschnitt dieses Durchgangs (72) auf wenigstens einem Teil seiner Länge fortschreitend auf die Querwand (90, 96) zu abnehmend ausgebildet ist.

3. Injektionsspritze nach Anspruch 2, **dadurch gekennzeichnet, dass** das hintere Ende (22B) der Nadel anfänglich in dem Durchgang (72) angeordnet ist.

4. Injektionsspritze nach einem der vorhergehenden Ansprüche, **dadurch gekennzeichnet, dass** der Führungskörper (66) Mittel zur Zentrierung (44, 46, 70) dieses Körpers in Bezug auf die Achse (X-X) der Nadel (22) aufweist, wobei diese Mittel zur Zentrierung anfänglich mit der inneren Seitenwand des Spritzenkörpers (12) in der Umgebung des hinteren Endes (22B) der Nadel zusammenwirken.

5. Injektionsspritze nach Anspruch 4, **dadurch gekennzeichnet, dass** der Führungskörper (66) längs der Nadel (22) verschiebegeführt angeordnet ist.

6. Injektionsspritze nach einem der vorhergehenden Ansprüche, **dadurch gekennzeichnet, dass** das hintere Ende der Nadel vollständig von dem starren Körper (64) zur Führung überdeckt wird und hinter ihm zurückliegend angeordnet ist.

7. Injektionsspritze nach den Ansprüchen 5 und 6 zusammen, **dadurch gekennzeichnet, dass** die Stützelemente (40) außen an ihren freien Enden Vorsprünge (44) aufweisen, welche zur Aufnahme in einer in der Seitenwand des Spritzenkörpers (12) eingebrachten Vertiefung (102) ausgebildet sind, wenn sich das Schutzelement (36) in seiner aktiven Schutzstellung befindet, und der Führungskörper (66) zwischen den Stützelementen (40) des Schutzelementes angeordnet ist und mit diesem bis in eine Position, in der er unbeweglich gemacht worden ist, gegen einen Anschlag gegen den Spritzenkörper verschoben ist, und dass die Stützelemente (40) Nockenflächen (52) aufweisen, welche mit dem Führungskörper (66) zusammenwirken, um, wenn dieser unbeweglich gemacht worden ist, die Enden der Stützelemente (40) durch axiale Verstellung des Schutzelementes (36) der Nadel während der letzten Phase des Einschiebens des Betätigungskolbens (14) in den Spritzenkörper (12) voneinander zu beabstanden, wobei der Führungskörper (66) einen sicheren Rückhalt der Vorsprünge (44) in der Vertiefung (102) sicherstellt.

8. Injektionsspritze nach einem der vorhergehenden Ansprüche, **dadurch gekennzeichnet, dass** das Schutzelement (36) und der Spritzenkörper (12) korrespondierende Profilierungen (44, 102) als Vorsprung und Vertiefung zum Halten des Schutzelementes (36) in seiner aktiven Schutzstellung aufweisen, und dass der starre Körper (64) zur axialen Führung Mittel (66) zum sicheren Rückhalt der Verbindung dieser korrespondierende Profilierungen (44, 102) als Vorsprung und Vertiefung aufweist, wenn das Schutzelement (36) sich in seiner aktiven Schutzstellung befindet.

9. Injektionsspritze nach einem der vorhergehenden Ansprüche, **dadurch gekennzeichnet, dass** die Querwand (90) von dem Betätigungskolben (14) getragen wird.

10. Injektionsspritze nach einem der Ansprüche 1 bis 9, **dadurch gekennzeichnet, dass** die oder eine Querwand von einem durchstoßbaren Zwischenkolben (94) getragen wird, welcher zwischen dem hinteren Ende (22B) der Nadel und dem Betätigungskolben (14) angeordnet ist, wobei der Zwischenkolben (94) mit dem Betätigungskolben (14) ein Behältnis für eine Injektionsflüssigkeit (92) festlegt.

11. Anordnung für einen Nadelhalter bestehend aus einer Wand (20), die mit einer Injektionsnadel (22) fest verbunden ist, welche diese Wand (20) durchdringt, sowie aus einem beweglichen Schutzelement (36) für das Injektionsende (22A) der Nadel, welches in Bezug auf die Nadel zwischen einer eingeschobenen Position bei zurückliegendem Injektionsende (22A) der Nadel und einer aktiven Schutzstellung, in welcher sich das vordere Ende des Schutzelementes vor dem Injektionsende der Nadel befindet, verschiebbar angeordnet ist, und dieses Schutzelement Stützelemente (40) aufweist, welche durch die Wand (20) hindurch reichen und sich längs der Nadel (22) erstrecken, **dadurch gekennzeichnet, dass** sie einen starren Körper (64) zur axialen Führung des hinteren Endes der Nadel aufweist, wobei der starre Körper (64) zur axialen Führung von den freien Enden der Stützelemente (40) des Schutzelementes getragen wird.

12. Anordnung für einen Nadelhalter nach Anspruch 11, **dadurch gekennzeichnet, dass** das hintere Ende der Nadel vollständig von dem starren Körper (64) zur Führung überdeckt wird und hinter ihm zurückliegend angeordnet ist.

## Claims

1. Injection syringe (10) comprising on the one hand an elongated syringe body (12) consisting of a tube (16) and a front wall (20) provided with an injection needle (22), and on the other hand an operating rear piston (14) fitted so as to be movable in the tube (16), and in which the injection needle (22) extends axially inside the syringe body (12) up to a rear end (22B), away from which there is initially arranged, in the syringe body (12), at least one transverse wall (90, 96) which can be perforated, the needle (22) and the said transverse wall (90, 96) being movable in relation to each other from an initial position in which the transverse wall (90, 96) is at some distance from the needle (22) up to a final position in which the transverse wall (90, 96) is pierced by the said needle (22), the syringe having a movable guard (36) for the injection end (22A) of the needle, movable in relation to the body (12) under the effect of the operating piston (14) being pressed into the body (12), between a retracted position in the body withdrawn from the injection end (22A) of the needle and an active protection position in which the front end of the guard is forward of the injection end (22A) of the needle, this guard having jambs (40) which go through the syringe body (12) and partly extend into the body (12) along the said needle (22), **characterised in that** it has a rigid part (64) for axially guiding the rear end (22) of the needle when the transverse wall (90, 96) is perforated, this axial guiding rigid part (64) being carried by the free ends of the jambs (40) of the guard.

2. Syringe according to Claim 1, **characterised in that** there is a passage (72) in this axial guiding rigid part (64) for guiding the rear end (22B) of the needle, the section of the said passage (72) decreasing gradually over at least part of its length towards the said transverse wall (90, 96).

3. Syringe according to Claim 2, **characterised in that** the rear end (22B) of the needle is initially arranged in the said passage (72).

4. Syringe according to any one of the previous claims, **characterised in that** the said guiding part (66) comprises means (44, 46, 70) for centering the said part in relation to the centreline (X-X) of the needle (22), these centering means working in conjunction initially with the inside lateral wall of the syringe body (12) in the vicinity of the rear end (22B) of the needle.

5. Syringe according to Claim 4, **characterised in that** the said guiding part (66) is fitted so that it can move along the needle (22).

6. Syringe according to any one of the previous claims, **characterised in that** the rear end of the needle is completely covered by the said rigid guiding part (64) and is withdrawn from it.

7. Syringe according to Claims 5 and 6 taken together, **characterised in that** the jambs (40) have on the outside, at their free end, projections (44) suitable for being taken in a hollow (102) made in the lateral wall of the body (12) when the guard (36) is in the active protective position, the guiding part (66) being arranged between the jambs (40) of the guard and movable with it to an immobilisation position abutting against the syringe body, and **in that** the jambs (40) have cam surfaces (52) working in conjunction with the guiding part (66) when the latter is immobilised, in order to distance the ends of the jambs (40) by axial movement of the needle guard (36) at the time of the final phase of pressing the operating piston (14) into the body (12), the guiding part (66) ensuring that the projections (44) are positively held in the hollow (102).

8. Syringe according to any one of the previous claims, **characterised in that** the said guard (36) and the said syringe body (12) have associated reliefs (44, 102) as projections and hollows for holding the guard (36) in its active protective position, and **in that** the said rigid part for axial guiding (64) has means (66) for positive retaining of the engagement of the said associated reliefs (44, 102) as projection and hollow when the guard (36) is in its active protection position.

9. Syringe according to any one of the previous claims, **characterised in that** the transverse wall (90) is carried by the operating piston (14).

10. Syringe according to any one of the Claims 1 to 9, **characterised in that** the transverse wall or one transverse wall (96) is carried by a pierceable intermediate piston (94) arranged between the rear end (22B) of the needle and the operating piston (14), the intermediate piston (94) delineating with the operating piston (14) a housing for a fluid (92) to be injected.

11. Needle holder assembly comprising a wall (20) integral with an injection needle (22) which goes through the said wall (20), as well as a moving guard (36) for the injection end (22A) of the needle, movable in relation to the needle between a retracted position withdrawn from the injection end (22A) of the needle and an active protective position in which the front end of the guard (36) is ahead of the injection end of the needle, this guard having jambs (40) going through the said wall (20) and extending along the said needle (22), **characterised in that** it comprises a rigid part (64) for axially guiding the rear end of the needle, this rigid part (64) for axial guiding being carried by the free ends of the jambs (40) of the guard.

12. Needle holder assembly according to Claim 11, **characterised in that** the rear end of the needle is completely covered by the said rigid guiding part (64) and is withdrawn from it.
